(19) Europäisches Patentamt
European Patent Office
Office européen des brevets

(11) Numéro de publication: **0 092 459 B1**

(12) **FASCICULE DE BREVET EUROPÉEN**

(45) Date de publication du fascicule du brevet: **26.11.86**

(51) Int. Cl.[4]: **C 07 D 471/04**, A 61 K 31/44 // (C07D471/04, 235:00, 221:00)

(21) Numéro de dépôt: **83400706.4**

(22) Date de dépôt: **07.04.83**

(54) **Imidazo(1,2-a)pyridines, leur préparation et leur application en thérapeutique.**

(30) Priorité: **21.04.82 FR 8206841**

(43) Date de publication de la demande: **26.10.83 Bulletin 83/43**

(45) Mention de la délivrance du brevet: **26.11.86 Bulletin 86/48**

(84) Etats contractants désignés: **AT BE CH DE FR GB IT LI LU NL SE**

(56) Documents cité: **GB-A-1 076 089**

(73) Titulaire: **SYNTHELABO, 58, rue de la Glacière, F-75621 Paris Cedex 13 (FR)**

(72) Inventeur: **Kaplan, Jean- Pierre, 20, rue Arnoux, F-92340 Bourg- la- Reine (FR)**
Inventeur: **George, Pascal, 39, rue Henri de Vilmorin, F-94400 Vitry- sur- Seine (FR)**
Inventeur: **Bernardon, Jean- Michel, 24, Domaine du Château, F-91380 Chilly- Mazarin (FR)**

(74) Mandataire: **Ludwig, Jacques, SYNTHELABO Service Brevets 58, rue de la Glacière, F-75621 Paris Cedex 13 (FR)**

**EP 0 092 459 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention concerne des dérivés d'imidazo [1,2-α] pyridine, leur préparation et leur application en thérapeutique.

Des imidazo [1,2-α] pyridines ont déjà été décrites dans la littérature, par exemple dans les brevets britanniques 991 589 et 1 076 089 et dans diverses publications.

Les composés de la présente invention répondent à la formule

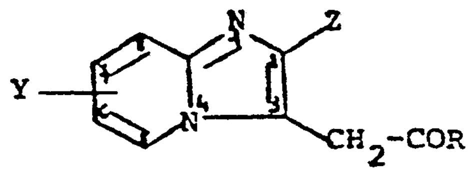

(I)

dans laquelle

. Y représente un atome d'hydrogène ou d'halogène ou un radical $C_{1\text{-}4}$alkyle,

. Z représente un radical furyle-2, thiényle-2 ou pyridyle-2 pouvant porter en position 5 un atome d'halogène ou un radical méthyle ou éthyle,

. R représente le radical $NR_1R_2$ dans lequel

$R_1$ et $R_2$ représentent chacun indépendamment l'un de l'autre soit un atome d'hydrogène, soit un radical $C_{1\text{-}5}$alkyle droit ou ramifié

Les composés de l'invention peuvent se présenter sous forme de base libre ou de sels d'addition à des acides pharmaceutiquement acceptables.

Les composés préférés de l'invention sont ceux dans lesquels $R_1$ et $R_2$ sont tous deux des atomes d'hydrogène ou des radicaux alkyles, et plus particulièrement parmi ceux-ci, les composés dans lesquels Y est en position 6 et représente soit un atome d'halogène, soit le radical méthyle, et Z représente un radical thiényle-2 substitué en 5 par un halogène ou un alkyle Selon l'invention, on peut préparer les composés (I) selon le schéma réactionnel suivant:

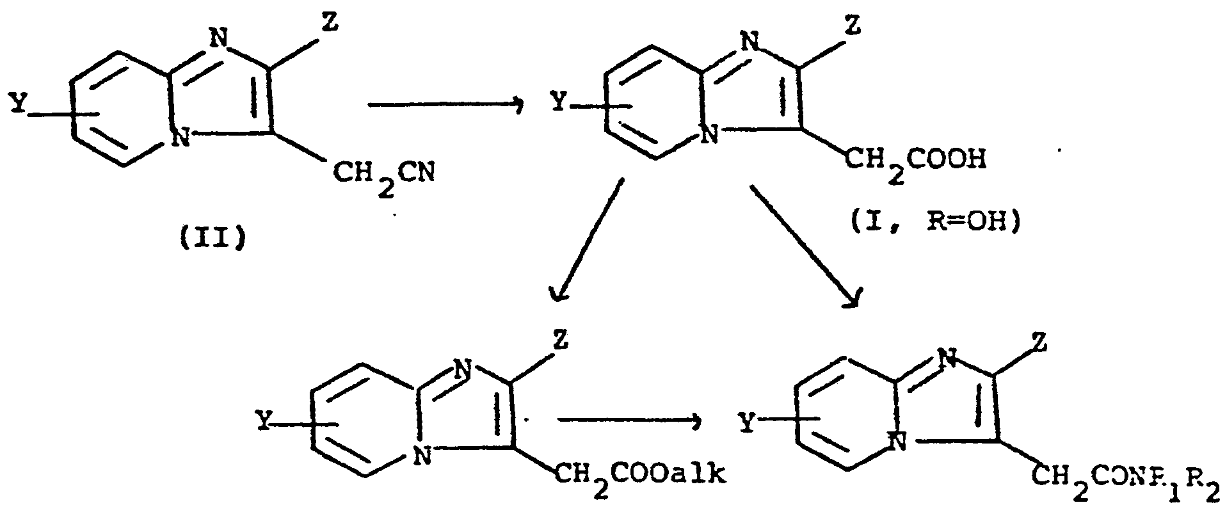

(II)

(I, R=OH)

(I, R=Oalk)

(I)

La réaction de transformation du nitrile (II) en acide est effectuée selon une méthode classique, par exemple à l'aide de potasse dans de l'éthanol à la température du reflux ou à l'aide d'acide acétique et d'acide chlorhydrique concentré à la température du reflux.

L'estérification de l'acide (I, R = OH) est réalisée selon toute méthode appropriée, par exemple par réaction de l'acide avec le chlorure de sulfonyle et l'alcool correspondant.

L'amidification est effectuge selon toute méthode appropriée, par exemple soit par réaction de l'acide (I, R = OH) avec le carbonyldiimidazole puis traitement par l'amine $HNR_1R_2$, soit par réaction de l'ester (I, R = Oalk) avec l'amine $HNR_1R_2$.

Les nitriles de départ II sont obtenus soit selon la méthode décrite dans la littérature, en particulier dans le brevet britannique 1 076 089, soit selon le schéma réactionnel suivant:

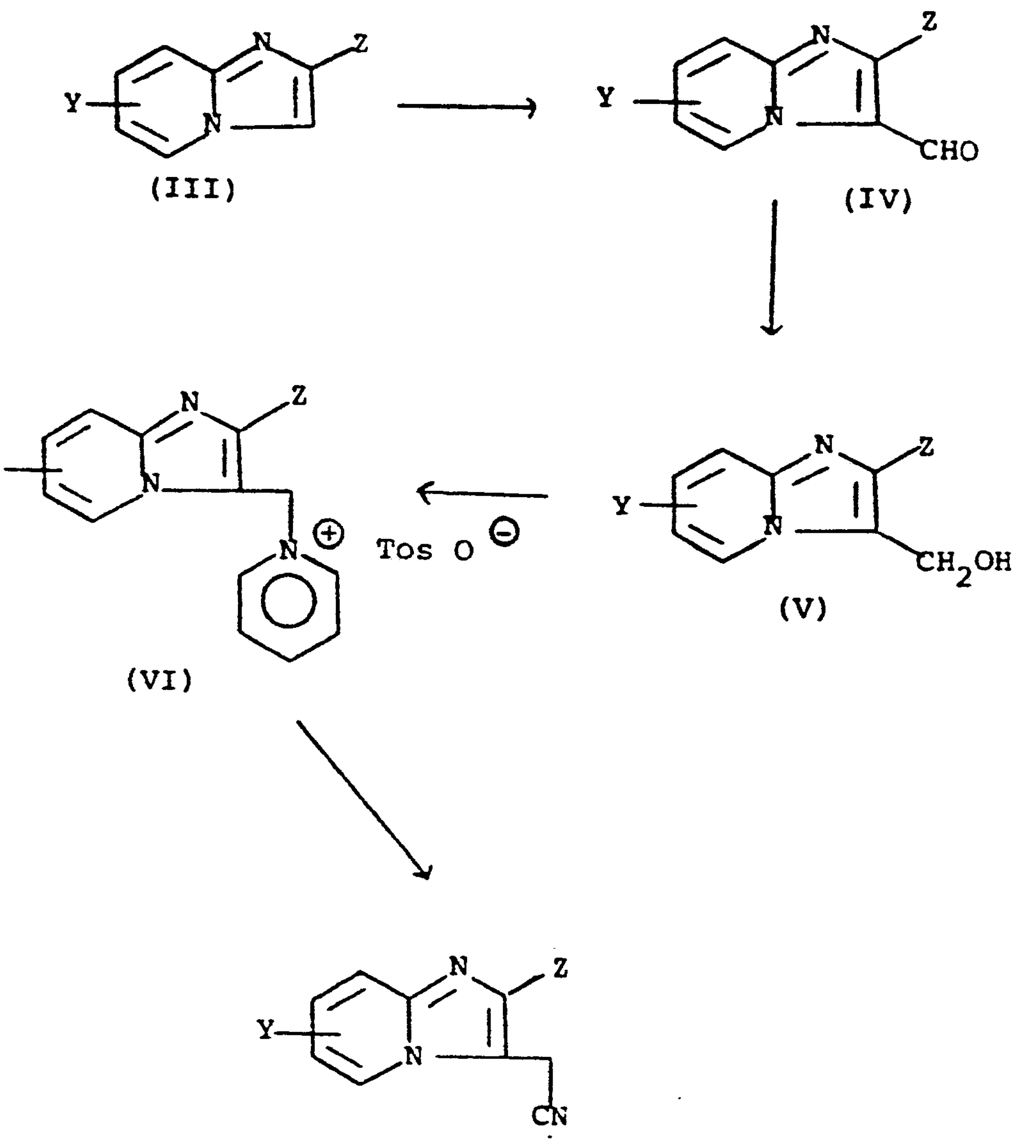

(III) (IV) (V) (VI) (II)

On part du composé (III) que l'on obtient par condensation d'une amino-2 pyridine substituée

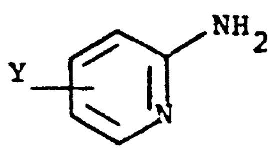

avec und α-bromo-cétone de formule $ZCOCH_2Br$.

On prépare ensuite l'aldéhyde (IV), selon toute méthode appropriée, par exemple par formylation du composé (III) à l'aide de chlorure de dimethylformamide. On réduit l'aldéhyde (IV) en alcool (V), par exemple à l'aide de borohydrure de sodium. On prépare le tosylate de pyridinium (VI) par tosylation de l'alcool (V) dans de la pyridine et enfin on effectue une cyanation en milieu aqueux du tosylate de pyridinium (VI).

Les exemples suivants illustrent l'invention.

Les analyses et les spectres IR et RMN confirment la structure des composés.

**Exemple 1**

Cloro-6 (pyridyl-2)-2 imidazo [1,2-α] pyridine-3-acétonitrile.

1. Dans un erlenmeyer, on introduit 200 g (0,711 mole) de bromhydrate de bromacétyl-2 pyridine, 91,5 g (0,711 mole) d'amino-2 chloro-5 pyridine, 179,4 g (2,1355 moles) de $NaHCO_3$ et 500 ml d'éthanol. On chauffe à 40°C progressivement et maintient à cette température pendant 4 heures. On filtre le solide, l'exrait entre l'eau et le chloroforme, décante la phases organique, sèche sur sulfate de magnsésium et évapore. On fait recristalliser le composé obtenu dans de l'alcool isopropylique.

F = 190-191°C.

2. Dans un erlenmeyer, on introduit 165 ml de DMF, tout en refrodissant à -30°C, on ajoute goutte à goutte 50,77 g (0,4 mole) de chlorure d'oxalyle. On agite 30 mn à 0°C puis laisse revenir à la température ambiante. On ajoute par petites quantités 22,9 g (0,1 mole) de chloro-6 (pyridyl-2)-2 imidazo [1,2-α] pyridine, agite 6 heures à la température ambiante et laisse reposer la nuit. On jette le milieu réactionell dans 500 ml d'eau et l'alcalinise avec de l'ammoniac. On filtre le solide, le lave à l'eau puis à l'acétone. On le triture dans le méthanol bouillant.

F = 227-228°C.

3. Dans un ballon, on introduit 20 g (0,077 mole) de l'aldéhyde précédent dans 300 ml de méthanol. On ajoute goutte à goutte 1,45 g (0,0385 mole) de $NaBH_4$ dans 10 ml d'eau et agite 8 heures à la température ambiante. On évapore à sec, triture le résidu solide dans de l'eau, le filtre et le lave à l'eau. On extrait la chloro-6 hydroxyméthyl-3(pyridyl-2)-2 imidazo [l,2-a] pyridine au chloroforme, sèche la phase organique sur $MgSO_4$ et évapore. On triture le résidu dans de l'alcool isopropylique bouillant.

F = 200-201°C.

4. Dans un ballon, on introduit 5,2 g (0,02 mole) de l'alcool précédent et 50 ml de pyridine. On ajoute 4,2 g (0,022 mole) de chlorure de p-toluènesulfonyl et agite 8 heures à la température ambiante. On évapore à sec et triture le résidu dans de l'eau. On filtre le solide, le lave avec un minimum d'acétone et le sèche sur dessicateur.

F = 220-225°C.

5. Dans un erlenmeyer, on introduit 24,9 g (0,049 mole) du tosylate de pyridinium obtenu en 4, 7,2 g (0,147 mole) de NaCN et 300 ml d'eau. On chauffe à la température du reflux de 2 à 5 heures, refroidit la suspension, filtre le solide et le lave à l'eau. On le dissout ensuite dans du chloroforme, sèche la phase organique sur $MgSO_4$, évapore et fait recristalliser le nitrile dans de l'acétate d'éthyle.

F = 224-225°C.

**Exemple 2**

(Chloro-5 thiényl-2)-2 méthyl-6 imidazo [l,2-α] pyridine-3-acétonitrile.

1. Dans un erlenmeyer, on introduit 60 g (0,25 mole) de bromoacétyl-2 chloro-5 thiophène, 7,1 g (0,25 mole) de méthyl-5 amino-2 pyridine, 42 g (0,5 mole) de $NaHCO_3$ et 300 ml d'éthanol. On chauffe à 60°C pendant 5 heures. On évapore à sec, reprend le résidu d'évaporation entre l'eau et l'éther, filtre le solide et le sèche au dessicateur en présence de $P_2O_5$. On fait recristalliser le composé obtenu dans de l'alcool isopropylique.

F = 188-189°C.

2. Dans un ballon, on introduit 55 g (0221 mole) du composé obtenu sous 1 et 400 ml d'acide acétique. On ajoute, goutte à goutte, 37,4 g (0,331 mole) de diméthylamine à 40 % dans l'eau, refroidit à 0°C et ajoute, goutte à goutte, 24,8 g (0,247 mole) de formol à 30% dans l'eau. On agite 4 heures et laisse au repos une nuit. On évapore à sec, reprend le résidu dans de l'eau, alcalinise, extrait au chloroforme, décante la phase organique, sèche sur $MgSO_4$ et évapore. On reprend le solide par 200 ml de méthanol et ajoute 41,9 g (0,296 mole) d'iodure de méthyle. On agite 4 heures, laisse au repos une nuit, filtre l'iodure d'ammonium et sèche.

F = 200-205°C.

3. Dans un réacteur, on introduit 86,5 g (0,193 mole) du sel quaternaire obtenu sous 2, 28,4 g (0,579 mole) de NaCN et 1 litre d'eau. On chauffe à la température du reflux pendant 10 heures, filtre le solide, le lave à l'eau, le dissout dans du chloroforme, sèche la phase organique sur $MgSO_4$ et évapore le solvant. On fait recristalliser le nitrile obtenu dans l'acétate d'éthyle.

F = 167-169°C.

**Exemple 3**

Chloro-6 N,N-diméthyl (bromo-5 furyl-2)-2 imidazo [1,2-α] pyridine-3-acétamide.

[Y= 6-Cl, Z= Br 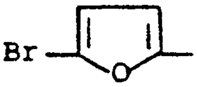 ,$NR_1R_2$=N$(CH_3)_2$]

1. Dans un ballon tricol de 2 1, on introduit 40,5 g (0,12 mole) de chloro-6 (bromo-5 furyl-2)-2 imidazo [1,2-α] pyridine-3-acétonitrile, 33,6g (0,6 mole) de potasse et 1 l d'èthanol. On chauffe le mélange réactionnel à la température du reflux pendant 10 heures, on l'évapore et le reprend par de l'eau puis on extrait au chloroforme. On acidifie jusqu'à pH 4,5, on filtre le solide et le lave à l'eau puis avec le minimum d'acétone et on le sèche sur $P_2O_5$.

F = 255-256° C.

2. On introduit, dans un erlenmeyer, 10 g (0,0281 mole) de l'acide obtenu sous 1 et 200 ml de tétrahydrofuranne. On ajoute, par petites quantités, 4,6 g (0,0281 mole) de carbonyldiimidazole et on agite le mélange réactionnel à la température ambiante pendant 5 heures. On introduit de la diméthylamine en excès et agite le mélange réactionnel pendant 4 heures. Après évaporation à siccité, on reprend le résidu par de l'eau on alcalinise à pH 11 et on filtre le solide. On le dissout dans du chloroforme, le lave à l'eau, le sèche sur sulfate de magnésium et évapore.

F = 252-253° C.

**Exemple 4**

Méthyl-6 (méthyl-5 thiényl - 2)-2 imidao [1,2-α] pyridine-3-acétamide.

1. Dans un erlenmeyer on introduit 5,4 g (0,02 mole) de méthyl-6 (méthyl-5 thiényl-2)-2 imidazo [1,2-α] pyridine-3-acétonitrile, 11,2 g (0,2 mole) de potasse et 300 ml d'éthanol. On chauffe à la température du reflux pendant 10 heures. On évapore à siccité, dissout le résidu dans de l'eau et extrait au chloroforme. On décante la phase aqueuse, acidifie à pH4 et filtre le solide que l'on lave à l'eau et sèche en présence de $P_2O_5$. On fait recristalliser le produit dans du méthanol.

F = 230-231° C.

2. Dans un erlenmeyer on introduit 5 g (0,0174 mole) d'acide méthyl-6 (méthyl-5 thiényl-2)-2 imidazo [1,2-α] pyridine-3-acétique et 100 ml de tétrahydrofuranne. On ajoute, par petites quantités, 2,8 g (0,0174 mole) de carbonyldiimidazole et agite, à la température ambiante, pendant 4 heures. On introduit goutte à goutte 20 ml de tétrahydrofuranne saturé en ammoniac et on agite pendant 4 heures. Après évaporation à siccité, on reprend le résidu par de l'eau, alcalinise à pH 11; filtre le solide que l'on lave à l'eau et sèche sur $P_2O_5$. On le fait ensuite recristalliser dans de l'alcool isopropylique.

F = 236-237° C.

Selon le même schéma réactionnel, on a préparé à titre d'exemples les composés du tableau suivant.

## TABLEAU

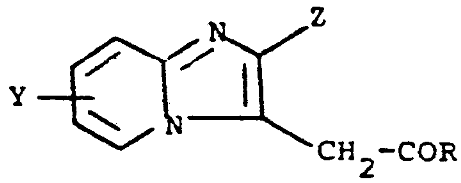

(I)

| Composé | Y | Z | R | F(°C) |
|---|---|---|---|---|
| 1 | 6-Cl | 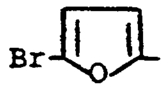 | $NH_2$ | 268-269 |
| 2 | 6-Cl | 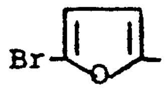 | $N(CH_3)_2$ | 252-253 |
| 3 | 6-$CH_3$ | 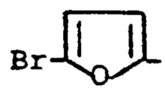 | $NH_2$ | 266-267 |
| 4 | 6-$CH_3$ | 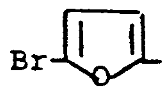 | $N(CH_3)_2$ | 250-251 |
| 5 | 6-$CH_3$ | 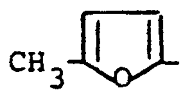 | $NH_2$ | 274-276 |
| 6 | 6-$CH_3$ | 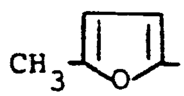 | $N(CH_3)_2$ | 220-221 |
| 7 | 6-$CH_3$ | 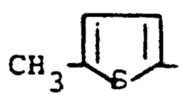 | $NH_2$ | 236-237 |
| 8 | 6-$CH_3$ | 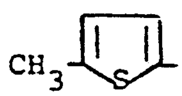 | $N(CH_3)_2$ | 185-186 |
| 9 | 6-$CH_3$ | 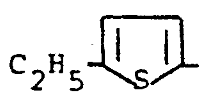 | $N(CH_3)_2$ | 156-157 |
| 10 | 6-Cl | 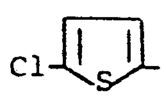 | $NH_2$ | 257-258 |
| 11 | 6-Cl | 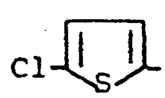 | $N(CH_3)_2$ | 196-197 |
| 12 | 6-Cl | 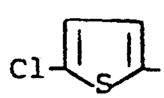 | $N(C_3H_7)_2$ | 143-144 |

TABLEAU (Suite)

| Composé | Y | Z | R | F(°C) |
|---|---|---|---|---|
| 13 | 6-$CH_3$ | 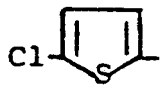 | $NH_2$ | 245-246 |
| 14 | 6-$CH_3$ | 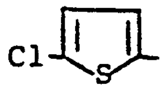 | $N(CH_3)_2$ | 197-198 |
| 15 | 6-Cl | 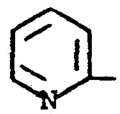 | $NH_2$ | 241-242 |
| 16 | 6-Cl | 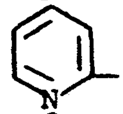 | $N(CH_3)_2$ | 226-227 |
| 17 | 6-Cl | 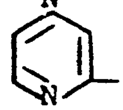 | $N(C_2H_5)_2$ | 189-190 |
| 18 | 6-$CH_3$ | 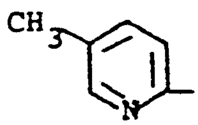 | $NH_2$ | 270-271 |
| 19 | 6-$CH_3$ | 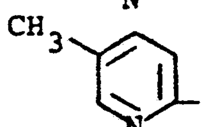 | $NHCH_3$ | 222-223 |
| 20 | 6-$CH_3$ | 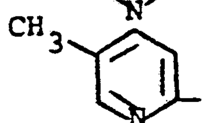 | $N(CH_3)_2$ | 219-220 |
| 21 | 6-$CH_3$ | 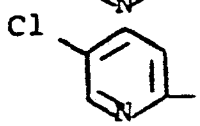 | $NH_2$ | 264-265 |

Les nitriles de départ (II) sont des composés nouveaux et font partie de l'invention. Les composés préparés à titre d'exemples sont représentés dans le tableau II suivant.

## TABLEAU II

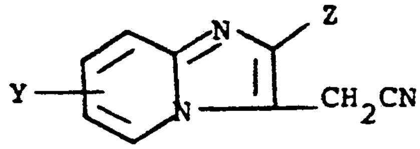

| Composé | Y | Z | F (°C) |
|---|---|---|---|
| 1 | 6-Cl | 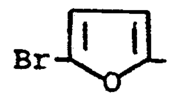 | 217-219 |
| 2 | 6-$CH_3$ | 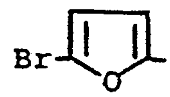 | 215-217 |
| 3 | 6-$CH_3$ | 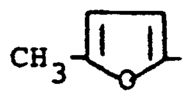 | 217-218 |
| 4 | 6-$CH_3$ | 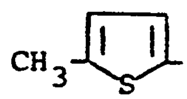 | 185-186 |
| 5 | 6-$CH_3$ | 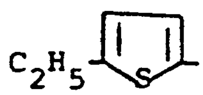 | 195-196 |
| 6 | 6-Cl | 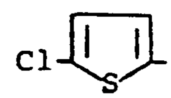 | 202-204 |
| 7 | 6-$CH_3$ | 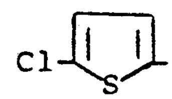 | 167-169 |
| 8 | 6-Cl | 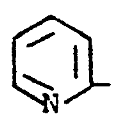 | 224-225 |
| 9 | 6-$CH_3$ | 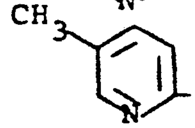 | 196-197 |
| 10 | 6-$CH_3$ | 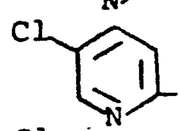 | 232-233 |
| 11 | 6-Cl | 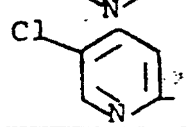 | 226-227 |

Les composés de l'invention ont été soumis à des essais pharmacologiques qui ont montré leurs intéressantes propriétés pharmacologiques dans divers domaines.

La toxicité des composés à été déterminée chez la souris par voie intrapéritonéale.

La DL 50 va de 500 à 100Omg/kg.

L'activité anxiolytique à été déterminée selon le "eating test" (Stephens, R.J. (1973) Brit. J. Pharmac., 49, 146 P).

Dans ce test, les doses qui augmentent la consommation alimentaire des souris varient de 1 à 30 mg/kg, i.p.

L'activité des composés dans le domaine de la circulation cérébrale ont été déterminée dans le test de l'hypoxie hypobare.

Des souris de souche CDI sont maintenues dans une atmosphère appauvrie en oxygène, par réalisation d'un vide partiel (190 mm de mercure correspondant à 5,25% d'oxygène).

Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement. La DAM vade 0,3 à 32 mg/kg i.p.

L'activité anticonvulsivante a été déterminée selon le test de l'antagonisme vis à vis de la mortalité induite par la bicuculline chez la souris (Worms, p.,Depoortere, H. and Lloyd, K.G. (1979) Life Sci., 25, 607-614). Les produits à étudier sont injectés par voie intrapéritonéale, 30 mn avant la bicuculline (0,9 mg/kg i.v). Le critère retenu pour ce test étant la léthalité, les pourcentages de mortalité sont notés pour chaque lot, 2 heures après administration de la bicuculline (lot témoin: 100% mortalité). Pour chague produit la dose active 50% (DA 50 ou dose protégeant 50% d'animaux des effets léthaux de la bicuculline) est déterminée graphiquement. La DA 50 des composés de l'invention varie entre 1 à 30 mg/kg par voie i.p.

L'activité sédative ou hypnotique a été déterminée en observant l'action des composés sur l'ECoG du rat curarisé (Depoortere H., Rev. E.E.G. Neurophysiol., (1980) 10, 3, 207-214). Chez le rat curarisé, les produits à étudier ont été injectés par voie intrapéritonéale ou orale aux doses croissantes de 1 à 30 mg/kg. Ils induisent des tracés de sommeil à partir des doses allant de 1 à 10 mg/kg i.p ou p.o.

Les résultats de ces différents tests montrent que les composés de l'invention possèdent des propriétés anxiolytiques, antianoxiques, inductrices de sommeil, hypnotiques et anticonvulsivantes; les composés de l'invention sont utiles pour le traitement des états d'anxiétés, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens et pour le traitement des encéphalopathies métaboliques.

Les composés de l'invention peuvent être présentés sous toute forme appropriée pour l'administration par voie orale, ou parentérale, par exemple sous forme de comprimés, de dragées, de gélules, de solutions buvables ou injectables etc.. avec tout excipient approprié.

La posologie quotidienne peut aller de 0,5 à 2000 mg.

## Revendications

pour les Etats contractants BE, CH, DE, FR, GB, IT, LI, LU, NL, SE.

1. Imidazo [1,2-α] pyridines répondant à la formule (I)

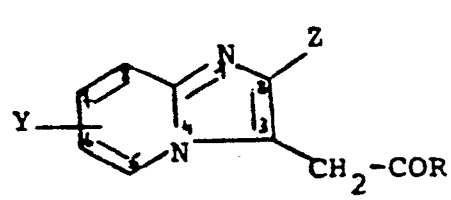

(I)

dans laquelle

. Y représente un atome d'hydrogène ou d'halogène ou un radical $C_{1-4}$alkyle,

. Z représente un radical furyle-2, thiényle-2 ou pyridyle-2 pouvant porter en position 5 un atome d'halogène ou un radical méthyle ou éthyle,

. R représente le radical $NR_1R_2$ dans lequel $R_1$ et $R_2$ représentent chacun indépendamment l'un de l'autre soit un atome d'hydrogène, soit un radical $C_{1-5}$ alkyle droit ou ramifié ainsi que leurs sels d'addition à des acides pharmaceutiquement acceptables.

2. Dérivés selon la revendication 1, dans lesquels $R_1$ et $R_2$ sont tous deux des atomes d'hydrogène ou des radiaux alkyles.

3. Dérivés selon la revendication 2, dans lesquels Y est en position 6 et représente soit un atome d'halogène

soit le radical méthyle.

4. Dérivés selon la revendication 3, dans lesquels Z représente un radical thiényle-2 substitué en 5 par un halogène ou un alkyle.

5. Procédé de préparation des composés selon la revendication 1, procédé caractérisé en ce que l'on transforme en acide le nitrile de départ (II)

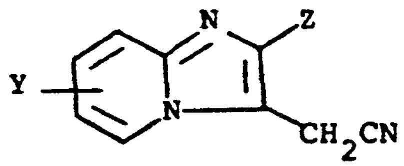

(II)

puis on estérifie ou amidifie l'acide et on transforme l'ester en amide (I); les radicaux ayant les significations données dans la revendication 1.

6. Médicament caractérisé en ce qu'il contient un composé tel que spécifié dans l'une quelconque des revendications 1 à 4.

7. Composition pharmaceutigue caractérisée en ce qu'elle contient un composé tel que specifié dans l'une quelconque des revendications 1 à 4 en association avec tout excipient approprié.

**Revendications**

pour l'Etat contractant AT.

1. Procédé de préparation d'imidazo [l,2-α] pyridines, sous forme de bases libres ou de sels d'addition à des acides pharmaceutiquement acceptables, répondant à la formule (I)

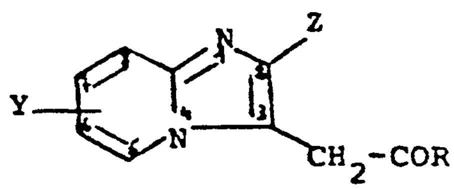

(I)

dans laquelle

. Y représente un atome d'hydrogène ou d'halogène ou un radical $C_{1-4}$alkyle,

. Z représente un radical furyle-2, thiényle-2 ou pyridyle-2 pouvant porter en position 5 un atome d'halogène ou un radical méthyle ou éthyle,

. R représente le radical $NR_1R_2$ dans lequel $R_1$ et $R_2$ représentent chacun indépendamment l'un de l'autre soit un atome d'hydrogène, soit un radical $C_{1-5}$ alkyle droit ou ramifié, procédé caractérisé en ce qu'on transforme en acide le nitrile de départ (II)

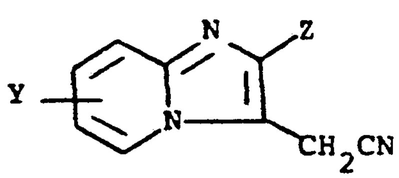

(II)

puis on estérifie ou amidifie l'acide et on transforme l'ester en amide (I), les radicaux ayant les significations données ci-dessus.

2. Procédé selon la revendication 1, caractérisé en ce que, dans la formule (I), $R_1$ et $R_2$ sont tous deux des atomes d'hydrogène ou des radicaux alkyles.

3. Procédé selon la revendication 2, caractérisé en ce que, dans les formules (I) et (II), Y est en position 6 et représente soit un atome d'halogène soit le radical méthyle.

4. Procédé selon la revendication 3, caractérisé en ce que, dans les formules (I) et (II), Z représente un radical thiényle-2 substitué en 5 par un halogène ou un alkyle.

**Patentansprüche**

für die Vertragsstaaten BE, CH, DE, FR, GB, IT, LI, LU, NL, SE

1. Imidazo [1,2-α]-pyridine der Formel (I)

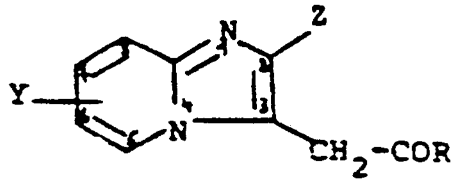

(I)

in welcher Y ein Wasserstoff- oder ein Halogenatom oder einen $C_{1-4}$-Alkylrest bedeutet, Z für einen 2-Furyl-, 2-Thienyl- oder 2-Pyridylrest steht, der in der 5-Stellung ein Halogenatom oder einen Methyl- oder Ethylrest enthalten kann, R den Rest $NR_1R_2$ darstellt, in welchem $R_1$ und $R_2$ jeweils unabhängig voneinander entweder für ein Wasserstoffatom oder für einen geradkettigen oder verzweigten $C_{1-5}$-Alkylrest steht, sowie deren Additionssalze mit pharmazeutisch verwendbaren Säuren.

2. Derivate nach Anspruch 1, in welchem $R_1$ und $R_2$ beide für Wasserstoffatome oder Alkylreste stehen.

3. Derivate nach Anspruch 2, in welchen Y in 6-Stellung steht und entweder ein Wasserstoffatom oder den Methylrest bedeutet.

4. Derivate nach Anspruch 3, in welchen Z einen in 5-Stellung durch ein Halogen oder ein Alkyl substituierten 2-Thienylrest bedeutet.

5. Verfahren zur Herstellung von Verbindungen nach Anspruch 1, dadurch gekennzeichnet, daß man das Ausgangsnitril der Formel (II)

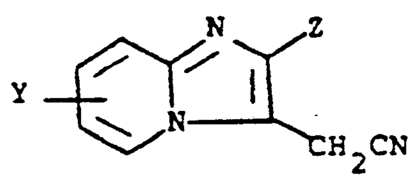

(II)

in Säure umwandelt, dann dieselbe verestert oder amidiert bzw. den Ester in das Amid (I) umwandelt, wobei die Reste die im Anspruch 1 angegebene Bedeutung haben.

6. Heilmittel, dadurch gekennzeichnet, daß es eine Verbindung enthält, wie sie in irgendeinem der Ansprüche 1 bis 4 dargelegt wurde.

7. Pharmazeutische Zusammensetzung, dadurch gekennzeichnet, daß sie eine Verbindung nach einem der Ansprüche 1 bis 4 in Kombination mit irgendeinem geeigneten Trägermaterial enthält.

**Patentansprüche**

Für den vertragsstaat: AT

1. Verfahren zur Herstellung von Imidazo [1,2- α]-pyridinen in Form der freien Basen oder der Additionssalze mit pharmazeutisch verwendbaren Säuren, die der Formel (I)

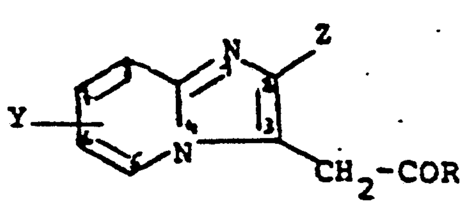

(I)

entsprechen, in welcher Y ein Wasserstoff- oder ein Halogenatom oder einen $C_{1-4}$-Alkylrest, Z einen gegebenenfalls in 5-Stellung durch ein Halogenatom oder einen Methyl- oder Ethylrest substituierten 2-Furyl-, 2-Thienyl- oder 2-Pyridylrest bedeutet, R für den Rest $NR_1R_2$ steht, in welchem $R_1$ und $R_2$ jeweils unabhängig voneinander entweder ein Wasserstoffatom oder einen geradkettigen oder verzweigten $C_{1\ 5}$-Alkylrest bedeutet, dadurch gekennzeichnet, daß man das Ausgangsnitril der Formel (II)

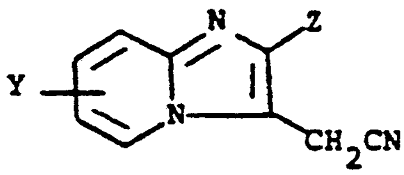

(II)

in die Säure umwandelt, dann dieselbe verestert oder amidiert bzw. den Ester in das Amid (I) umwandelt, wobei die Reste die oben genannte Bedeutung haben.

2. Verfahren nach Anspruch 1, dadurch gekennzeichnet, daß in der Formel (I) $R_1$ und $R_2$ beide für Wasserstoffatome oder Alkylreste stehen.

3. Verfahren nach Anspruch 2, dadurch gekennzeichnet, daß in diesen Formeln (I) und (II) Y in 6-Stellung steht und entweder ein Halogenatom oder den Methylrest bedeutet.

4. Verfahren nach Anspruch 3, dadurch gekennzeichnet, daß in den Formeln (I) und (II) Z einen in 5-Stellung durch ein Halogen oder ein Alkyl substituierten 2-Thienylrest bedeutet.

Der Patentanwalt:

**Claims**

for the contracting States: BE, CH, DE, FR, GB, IT, LI, LU, NL, SE.

1. Imidazo [1,2-a] pyridines responding to formula (I)

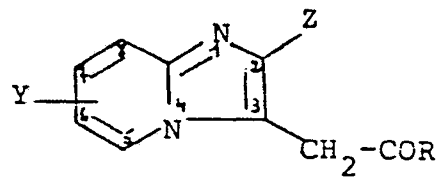

(I)

wherein

. Y denotes a hydrogen or halogen atom or a $C_{1-4}$alkyl radical,

. Z denotes a 2-furyl, 2-thienyl or 2-pyridyl radical optionally substituted in the 5-position by a halogen atom or a methyl or ethyl radical,

. R denotes a $NR_1R_2$ radical wherein $R_1$ and $R_2$ independently of one another each denote either a hydrogen atom or a linear or branched $C_{1-5}$alkyl radical,

and their pharmaceutically acceptable acid addition salts.

2. Derivatives according to claim 1, wherein both of $R_1$ and $R_2$ are hydrogen atoms or alkyl radicals.

3. Derivatives according to claim 2, wherein Y is in the 6-position and denotes either a halogen atom either the methyl radical.

4. Derivatives according to claim 3, wherein Z denotes a 2-thienyl radical substituted in the 5-position by a halogen or an alkyl.

5. A process for the preparation of the compounds according to claim 1, characterized in that the starting nitrile (II)

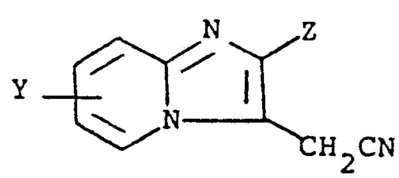

(II)

is converted into the acid, then said acid is esterified or amidified and the ester is converted into the amide (I), the radicals being as defined in claim 1.

6. A medicament characterized in that it contains a compound as specified in anyone of claims 1 to 4.

7. A pharmaceutical composition characterized in that it contains a compound as specified in anyone of claims 1 to 4, in combination with any suitable carrier.

**Claims**

for the contracting State: AT

1. A process for the preparation of imidazo [1,2-a] pyridines, in form of free bases or addition salts with pharmaceutically acceptable acids, responding to formula (I)

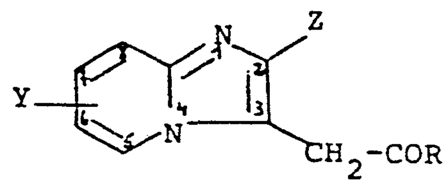

(I)

wherein

. Y denotes a hydrogen or halogen atom or a $C_{1-4}$alkyl radical,

. Z denotes a 2-furyl, 2-thienyl or 2-pyridyl radical optionally substituted in the 5 position by a halogen atom or a methyl or ethyl radical,

. R denotes a $NR_1R_2$ radical wherein $R_1$ and $R_2$ independently of one another each denote either a hydrogen atom or a linear or branched $C_{1-5}$alkyl radical,

process characterized in that the starting nitrile (II)

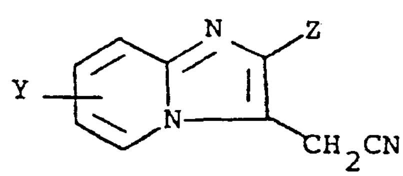

(II)

is converted into the acid, then said acid is esterified or amidified and the ester is converted into the amide (I), the radicals being as defined above.

2. A process according to claim 1, characterized in that, in formula (I), both of $R_1$ and $R_2$ are hydrogen atoms or alkyl radicals.

3. A process according to claim 2, characterized in that, in formulae (I) and (II), Y is in the 6-position and denotes either a halogen atom or the methyl radical.

4. A process according to claim 3, characterized in that, in formulae (I) and (II), Z denotes a 2-thienyl radical substituted in the 5-position by a halogen or an alkyl.